(19) Europäisches Patentamt
European Patent Office
Office européen des brevets

(11) EP 2 628 395 A1

(12) EUROPEAN PATENT APPLICATION

(43) Date of publication:
21.08.2013 Bulletin 2013/34

(51) Int Cl.:
A23L 1/00 (2006.01)
A23L 1/03 (2006.01)
A23L 1/0534 (2006.01)
A61K 8/00 (2006.01)
C11D 17/00 (2006.01)

(21) Application number: 12155410.9

(22) Date of filing: 14.02.2012

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(71) Applicant: Unilever N.V.
3013 AL Rotterdam (NL)

(72) Inventors:
- CAO, Jian
  200335 Shanghai (CN)
- Jin, Huajin
  200335 Shanghai (CN)
- Ma, Shouwei
  200335 Shanghai (CN)
- Zhou, Weizheng
  200335 Shanghai (CN)
- de Groot, Petrus Wilhelmus N.
  3133 AT Vlaardingen (NL)
- Pelan, Edward George
  3133 AT Vlaardingen (NL)
- Stoyanov, Simeon Dobrev
  3133 AT Vlaardingen (NL)
- Li, De
  Shanghai (CN)

(74) Representative: Fijnvandraat, Arnoldus
Unilever N.V.
Unilever Patent Group
Olivier van Noortlaan 120
3133 AT Vlaardingen (NL)

(54) **Aerated composition comprising ethylcellulose particles and cationic polymer**

(57) The present invention relates to an aqueous aerated composition comprising ethylcellulose particles. Moreover the present invention relates to a method for preparation of such aerated composition. The invention provides an aerated composition comprising ethylcellulose particles further comprising a cationic polymer, that improves stability of the aerated composition.

EP 2 628 395 A1

## Description

### FIELD OF THE INVENTION

The present invention relates to an aqueous aerated composition comprising ethylcellulose particles. Moreover the present invention relates to a method for preparation of such aerated composition.

### BACKGROUND TO THE INVENTION

Aerated food products or foams are generally known, and such foods include frozen and chilled food products, such as ice cream, mousses, and whipped cream. Gases commonly used for 'aeration' include air, nitrogen and carbon dioxide.

Also in personal care products foams are well known, for example in shaving foams and creams, as well as hair care products.

Two factors are of importance in the development of aerated products, and these are (i) the foamability of the product while introducing gas into the product during manufacture and (ii) the foam stability during storage, which is whether the gas bubbles tend to coalesce or collapse and whether the foam volume is retained during storage. Many additives are known to be included in the creation of stable foams, and these generally are compounds which are present on the gas bubble surface, which means on the gas-liquid interface during manufacturing of the foam. Known additives include proteins such as sodium caseinate and whey, which are highly foamable, and biopolymers, such as carrageenans, guar gum, locust bean gum, pectins, alginates, xanthan, gellan, gelatin and mixtures thereof, which are good stabilisers.

WO 2008/019865 A1 discloses aqueous foams and food products containing these. The gas bubbles in the foam are stabilised by interfacially-active particles (e.g. proteins), which are considered to be colloidal particles having a diameter between 0.5 nanometer up to several tens of a micrometer. Aerated food products are produced by mixing a preformed aqueous foam into a food product.

Plasari et al. (Trans IChemE, 1997, Vol 75, Part A, p. 237-244) disclose a process to precipitate ethylcellulose nanoparticles from a solution of ethanol, using water as nonsolvent, under various process conditions. The average particle size of the nanoparticles was reported to be from about 35 to about 100 nanometer, which partly aggregated into agglomerates having a size between 300 and 600 nanometer. The size of the nanoparticles was strongly dependent on the initial concentration of ethylcellulose in the solvent. The zeta-potential of the ethylcellulose nanoparticles was not determined, and will be about -40 mV, as the dispersion containing the ethylcellulose nanoparticles is not acidified, nor contains salt ions.

WO 2008/046699 A1 discloses aerated food products in the form of a stable foam, comprising 5-80 vol% gas bubbles, 15-90 wt% water and 0.001 to 10 wt% fibres, and further containing surface-active particles at the air-water interface. These aerated food products are very stable due to attractive interaction between the surface-active particles and the fibres. The fibers preferably have a length from 1 to 50 micrometer. These fibres may be made from materials like microcrystalline cellulose or citrus fibers, or waxy materials, like carnauba wax, shellac wax, or bees wax. The surface-active particles are preferably made from modified celluloses, modified starches, and insoluble proteins. They are preferably present in an amount between 0.001 and 10 wt%, and preferably have a volume weighted mean diameter between 0.01 and 10 micrometer, more preferably between 0.1 and 1 micrometer. In the examples a combination of microcrystalline cellulose fibers and ethylcellulose particles is disclosed.

WO 2009/033592 A1 discloses foams used for foods, wherein the foam may comprise ethylcellulose and a hydrocolloid.

WO 2006/067064 A1 discloses a shelf stable mousse, containing a hydrocolloid as foam stabiliser. The hydrocolloids are water-soluble and may be carboxymethyl cellulose or other cellulose derivatives like methyl cellulose, hydroxypropyl cellulose.

WO 2007/038745 A1 discloses cream compositions containing cellulose derivatives, e.g. ethyl cellulose, at a concentration up to about 0.15%, and water-soluble or water-swellable hydrocolloids (e.g. chitosan, gelatin) as stabiliser. These compositions can be used in producing whipped food compositions.

WO 2010/121490 A1 discloses a method to produce foams stabilised by ethylcellulose colloidal particles. The method involves a step to lower the pH or increase the ionic strength of a dispersion of ethylcellulose particles, in order to give the particles the right surface charge before foaming.

B.S. Murray et. al. (Food Hydrocolloids, 2011, vol. 25, p. 627-638) describe stabilisation of foams and emulsions using surface active particles, including cellulose and ethylcellulose complexes, hydrophobically modified starch granule. Proteins can be used to stabilize foams or emulsions, and the authors indicate that there is little evidence of attractive interactions between the particles and the proteins, so a possible explanation of the increased stability is that the proteins increase the accumulation of particles at the interface, giving rise to increased jamming of particles at the interface.

## SUMMARY OF THE INVENTION

Current methods for preparing aerated food products often have the disadvantage that the foams are not stable enough to be used in a food product which upon storage remains stable for at least a month, preferably several months. The total foam volume may decreases due to collapse of bubbles, or the gas bubble size increases due to processes like coalescence. Both processes lead to changing texture of the food product. Moreover, some systems have the disadvantage that several additives are required to stabilise the foams which do not provide nutritional value. Moreover such compounds may be expensive, or not compatible for food use. Further, many of the ingredients used to stabilise the gas phase in aerated food products need to be added at fairly high levels which can have deleterious textural and/or caloric consequences.

Hence there is a need for efficient stabilisation of foams that are suitable for use in food products or personal care products, while the stabiliser preferably is a cheap and commonly available raw material. Preferably the foams have high overruns and relatively small, uniformly sized gas bubbles. Moreover the use of only a low concentration of stabiliser in the food product would be advantageous. Such foams can be used in food products to create a favourable mouthfeel, like a creamy mouthfeel, without having high caloric value of food products which normally have a smooth and creamy mouthfeel.

We have now found that stable foams can be produced containing ethylcellulose particles as a stabiliser for the gas bubbles, further containing a cationic polymer. The use of the cationic polymer leads to improved stability of the foams. This leads to the creation of very stable foams which can be used to produce stable aerated food products.

Accordingly in a first aspect the invention provides an aqueous aerated composition comprising ethylcellulose particles and a cationic polymer,
wherein the ethylcellulose particles have a volume weight average diameter d4,3 between 50 and 500 nanometer, and wherein at least part of the ethylcellulose particles and cationic polymer is present at the gas bubble surface.

In a second aspect the invention provides A method for preparation of a composition according to the first aspect of the invention, comprising the steps:

(a) dispersing ethylcellulose particles having a volume weight average diameter d4,3 between 50 and 500 nanometer in an aqueous composition;
(b) addition of cationic polymer to the dispersion from step (a); and
(c) aerating the composition from step (b).

In a second aspect the invention also provides A method for preparation of a composition according to the first aspect of the invention, comprising the steps:

(a) dispersing ethycellulose particles having a volume weight average diameter d4,3 between 50 and 500 nanometer in water at a pH of 4 or lower or at an ionic strength of at least 20 millimolar;
(b) aerating the composition from step (a); and
(c) addition of cationic polymer to the dispersion from step (b).

## DETAILED DESCRIPTION OF THE INVENTION

Unless defined otherwise, all technical and scientific terms used herein have the same meaning as commonly understood by one of ordinary skill in the art.

All percentages, unless otherwise stated, refer to the percentage by weight, with the exception of percentages cited in relation to the overrun. In case a range is given, the given range includes the mentioned endpoints.

In the context of the present invention, the average particle diameter or average oil droplet diameter or average gas bubble diameter is expressed as the d4,3 value, which is the volume weighted mean diameter, unless stated otherwise. The volume based particle or droplet or bubble size equals the diameter of the sphere that has same the same volume as a given particle or droplet or bubble.

The term 'aerated' means that gas has been intentionally incorporated into a composition, for example by mechanical means. The gas can be any gas, but is preferably, in the context of food products, a food-grade gas such as air, nitrogen, nitrous oxide, or carbon dioxide. Hence the term 'aeration' is not limited to aeration using air, and encompasses the 'gasification' with other gases as well. The extent of aeration is measured in terms of 'overrun', which is defined as:

$$overrun = \frac{\text{volume of aerated product - volume of initial mix}}{\text{Volume of initial mix}} \times 100\% \qquad (1)$$

where the volumes refer to the volumes of aerated product and unaerated initial mix (from which the product is made). Overrun is measured at atmospheric pressure.

After formation, a foam will be vulnerable to coarsening by mechanisms such as creaming, Ostwald ripening and coalescence. By creaming, gas bubbles migrate under the influence of gravity to accumulate at the top of a product. Ostwald ripening or disproportionation refers to the growth of larger bubbles at the expense of smaller ones. Coalescence refers to merging of air bubbles by rupture of the film in between them.

A stable foam or aerated food product in the context of the present invention is defined as being stable for at least 30 minutes, more preferred at least an hour, more preferred at least a day, even more preferred at least a week, and most preferred at least a month, and most preferred several months. A stable foam can be defined to be stable with regard to total foam volume, and/or gas bubble size, and looses maximally 20% of its volume during 1 month storage. On the other hand systems may exist which loose more than 20% of its volume during 1 month storage, which nevertheless are considered to have a good stability, as the stability of such foams is much better than comparative foams.

Such a foam can be produced by aerating a solution of interest using an aerolatte, kenwood mixer, or a BA Mixer, to a fixed overrun of for example 100%. The foam is then placed into a 100 mL measuring cylinder, stoppered, and stored at 5°C, and the foam volume measured over time. Foams of which the average bubble size strongly increases over time are regarded to be less stable than foams of which the average bubble size remains small over time.

*Aerated compositions*

In a first aspect the invention provides an aqueous aerated composition comprising ethylcellulose particles and a cationic polymer,
wherein the ethylcellulose particles have a volume weight average diameter d4,3 between 50 and 500 nanometer, and wherein at least part of the ethylcellulose particles and cationic polymer is present at the gas bubble surface.

The combination of ethylcellulose particles and cationic polymer leads to an effective stabiliser system for the gas bubbles.

The general structural formula of ethylcellulose is:

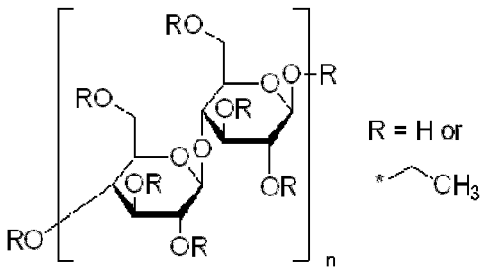

The degree of substitution of the ethylcellulose preferably used in the present invention is preferably between 2 and 3, more preferably about 2.5. The average number of hydroxyl groups substituted per anhydroglucose unit (the 'monomer') is known as the 'degree of substitution' (DS). If all three hydroxyls are replaced, the maximum theoretical DS of 3 results.

Suitable sources and types of the ethylcellulose preferably used in the present invention are supplied by for example Ashland Inc. (formerly Hercules), Sigma-Aldrich, and Dow Chemicals. Suitable ethylcellulose preferably has a viscosity between 5 and 300 cP at a concentration of 5 % in toluene/ethanol 80:20, more preferably between 100 and 300 cP at these conditions.

Preferably, the ethylcellulose particles have a volume weight average diameter d4,3 between 50 and 400 nanometer, more preferred between 50 and 300 nanometer, even more preferred between 50 and 200 nanometer, more preferred between 60 and 200 nanometer, and even more preferred between 70 and 200 nanometer. Most preferred the volume weighted mean diameter of the solid particles is between 100 and 200 nanometer, or even between 100 and 150 nanometer.

Preferably, at least 95% of the ethylcellulose particles has a diameter smaller than 500 nanometer, preferably smaller than 250 nanometer.

One of the advantages of an average particle size at the lower end of the indicated preferred ranges, is that the overrun reached can be higher than with particles at the higher end of the preferred ranges.

Preferably the composition has an overrun of at least 1%, preferably at least 5%. More preferred the overrun is at least 10%, preferably at least 30%, more preferred at least 50%, most preferred at least 100%. Preferably the overrun is maximally 400%, more preferred maximally 300%, or even maximally 200%. Preferably, at least 50% of the gas bubbles has a size smaller than 300 micrometer, preferably at least 75% of the gas bubbles has a size smaller than

300 micrometer. More preferred at least 50% of the gas bubbles has a size smaller than 100 micrometer, more preferred at least 75% of the gas bubbles has a size smaller than 100 micrometer.

The ethylcellulose particles may be prepared by any common process, for example an anti-solvent precipitation process as described in WO 2010/121490 A1.

Preferably the absolute value of the zeta-potential of the ethylcellulose particles is below 25 millivolt, preferably below 15 millivolt. The zeta-potential is a measure for the surface charge of a colloidal particle, and determines whether particle attract or repulse each other. At a relatively high absolute value of the zeta-potential the ethylcellulose particles are well dispersed, due to repulsion of the particles. This behaviour is observed at a zeta-potential of the particles having an absolute value above 25 millivolt. When the zeta-potential of the particles is decreased to an absolute value below 25 millivolt, the ethylcellulose particles aggregate, leading to the ethylcellulose particles being able to stabilise a foam.

An absolute value of the zeta-potential of the ethylcellulose particles smaller than 25 millivolt may be obtained by lowering the pH of a dispersion of ethylcellulose particles, or by increasing the salt content of such a dispersion, therewith increasing the concentration of ions in the dispersion. In the present case, the correct zeta-potential of ethylcellulose particles is obtained by the mixture of cationic polymer and ethylcellulose particles.

Preferably, in the aerated composition, the concentration of ethylcellulose particles ranges from 0.5% to 10% by weight, preferably from 1% to 5% by weight. More preferred the concentration ranges from 1 % to 4% by weight. In particular within these preferred ranges stable foams can be produced, although outside these preferred ranges also stable foams can be produced.

The composition of the invention contains a cationic polymer. Preferably the cationic polymer is chosen from the group consisting of poly-L-lysine, polydiallyldimethylammonium chloride, chitosan and gelatin.

Poly-L-lysine is a homopolymer consisting of a chain of the amino acid L-lysine.

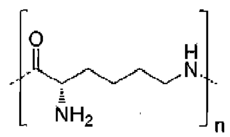

Molecular formula is $(C_6H_{12}N_2O)_n$. When dissolved in water, poly-L-lysine has an overall positive charge. Poly-L-lysine may be used as preservative for food products, and is used as such in Japan, Korea and USA especially. Polydiallyldimethylammonium chloride or polyDADMAC is a homopolymer of diallyldimethylammonium chloride (DADMAC). Molecular formula is $(C_8H_{16}NCl)_n$. When dissolved in water, polyDADMAC has an overall positive charge.

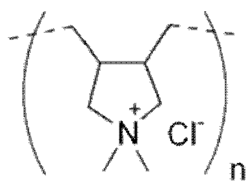

Chitosan is a linear polysaccharide composed of randomly distributed β-(1-4)-linked D-glucosamine (deacetylated unit) and N-acetyl-D-glucosamine (acetylated unit).

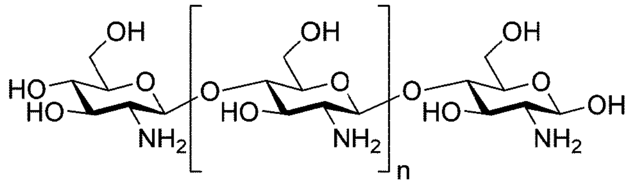

When dissolved in water, chitosan has an overall positive charge in an acidic solution, preferably at less than pH 5. Hence when the composition of the invention contains chitosan, then the pH of the composition preferably is lower than 5, preferably lower than 4.

The average molecular weight of chitosan is preferably at least 50,000 g/mol, preferably at least 100,000 g/mol, and preferably at least 250,000 g/mol.

Gelatin is a translucent, colorless, brittle (when dry), flavourless protein, derived from the collagen inside animals' skin and bones. It is commonly used as a gelling agent in a wide range of products such as food, pharmaceuticals, and

cosmetics. Type A gelatin refers to gelatin that has been acid treated during the manufacturing process, while type B gelatin refers to gelatin that has been alkali treated during the manufacturing process. Preferably, if gelatin is used, gelatin type A is used. In that case the pH of the foam preferably is lower than 7. The isoelectric point of gelatin type A is between about 7 and 9, and below this pH the gelatin type A has an overall positive charge. Type B gelatin has an isoelectric point between about 4.7 and 5.4, and below this pH the gelatin type B has an overall positive charge. If gelatin type B is used, the pH of a solution of gelatin is preferably below 4.7, in order to have a positively charged gelatin present.

Hence when the composition of the invention contains gelatin type A, then the pH of the composition preferably is lower than 7, preferably lower than 6, preferably lower than 5, preferably lower than 4. When the composition of the invention contains gelatin type B, then the pH of the composition preferably is lower than 4.7, preferably lower than 4. When using gelatin as cationic polymer, then preferably gelatin type A is used.

The molecular weight of gelatin influences stability of the foam, the higher the molecular weight, the higher the stability of the foam.

Preferably the cationic polymer used is chosen from the group consisting of poly-L-lysine, polydiallyldimethylammonium chloride, and chitosan. In case the aerated composition will be used as ingredient of a food product, then preferably chitosan and/or poly-L-lysine and/or gelatin is used as cationic polymer.

Preferably, the concentration of polymer ranges from 0.005% to 5% by weight of the composition. Preferably the concentration ranges from 0.05% to 5%, preferably from 0.5% to 4%.

Hence the weight ratio of ethylcellulose to cationic polymer ranges from 2000:1 to 1:10, preferably from 100:1 1 to 1:5, preferably from 10:1 to 1:1.

In case of chitosan, the weight ratio of ethylcellulose to chitosan preferably ranges from 50:1 1 to 5:1, preferably from 30:1 1 to 10:1.

In case of gelatin, the weight ratio of ethylcellulose to gelatin preferably ranges from 150:1 to 5:1, preferably from 100:1 1 to 10:1, more preferred from 100:1 1 to 30:1.

Preferably, the composition further comprises triphospate anions ($P_3O_{10}{}^{5-}$), preferably sodium triphosphate ($Na_5P_3O_{10}$).

Sodium triphosphate, $Na_5P_3O_{10}$, is the sodium salt of the polyphosphate penta-anion. It is contained in large number of household and industrial products.

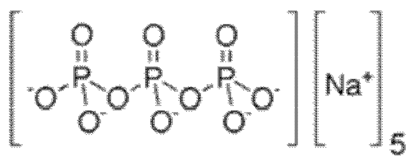

The advantage of the presence of triphosphate anions is that the stabilising system of ethylcellulose particles and cationic polymer is further enforced by the ionic interaction between the polymer and the triphosphate anions. The concentration of triphosphate anions preferably ranges from 0.01 to 2.0% by weight of the composition, preferably from 0.1 to 0.8% by weight.

The weight ratio of tripolyphosphate to cationic polymer preferably ranges from 10:1 1 to 1:2, preferably from 5:1 to 5:1, more preferred from 3:1 to 1:1.

Especially preferred combinations of ethylcellulose - cationic polymer and sodium triphosphate are:

- ethylcellulose, poly-L-lysine, and triphosphate
- ethylcellulose, polyDADMAC, and triphosphate
- ethylcellulose, chitosan, and triphosphate

When the composition contains gelatin as cationic polymer, then preferably the composition further comprises gum arabic. Gum arabic and gelatin are known to form complexes, which for instance can be used to coat oil droplets with a gelatin - gum arabic mixture (complex coacervation). Gum arabic is a hydrocolloid, that can be used as thickener in aqueous compositions, and mainly contains polysaccharides. Gum arabic is also known as acacia gum.

Preferably the weight ratio gelatin to gum arabic ranges from 0.75:1 to 1:0.75, preferably 0.9:1 to 1:0.9. More preferred the weight ratio of ethylcelluse : gelatin : gum arabic ranges from 2:1:1 1 to 10:1:1.

*Product comprising the aerated composition*

The present invention provides A food product or a personal care product or a home care product comprising the composition according to the first aspect of the invention.

An edible or a food product in the context of the present invention encompasses, but is not limited to, food products including beverages, dietetic foods, dietary supplements, pharmaceutic compositions, and others. The products may contain ingredients common in the art. Preferred food products according to the invention are oil-in-water emulsions like mayonnaise, light mayonnaises, dressings, and creams. Another preferred food product containing the aerated composition of the invention is an ice cream.

In the context of the present invention, a home care product is a product which is normally used for cleaning items such as hard surfaces in the home, or cleaning items such as the dishes and other kitchen hardware, or may be laundry detergents in liquid or solid form (powders, tablets), or may be laundry conditioners. Examples of such products are liquid or gel cleaners for the kitchen, bathroom, or toilet, and dishwashing liquid.

In the context of the present invention a personal care product is a product which is used by a consumer for personal cleaning, hygiene, and/or beauty. Cosmetic products in the context of the present invention encompass, but is not limited to, skin creams, body lotions, shampoos, hair conditioners, toothpastes, deodorants, hair styling products, personal soap bars, and liquid personal soaps.

*Method for preparation of aerated compositions*

In a second aspect the invention provides In a second aspect the invention provides A method for preparation of a composition according to the first aspect of the invention, comprising the steps:

(a) dispersing ethylcellulose particles having a volume weight average diameter d4,3 between 50 and 500 nanometer in an aqueous composition;
(b) addition of cationic polymer to the dispersion from step (a); and
(c) aerating the composition from step (b).

The pH of the dispersion in step (a) preferably ranges from 6 to 7, more preferred from 6.5 to 6.7. Alternatively the pH may be lower than 4. By the addition of the cationic polymer, the surface charge of the ethylcellulose particles is adjusted such that the absolute value of the zeta-potential of the ethylcellulose particles is lower than 25 millivolt, leading to the ethylcellulose becoming effective stabilisers of gas bubbles.

In step (b), the cationic polymer may be added to the dispersion of the ethylcellulose particles in pure form, or may be added as a solution or dispersion in water. In that case the pH of the dispersion or solution is such that the polymer has an overall positive charge.

Aeration in step (c) may be done by any commonly known method for aerating liquid compositions.

Alternatively, the aerated composition is prepared by A method for preparation of a composition according to the first aspect of the invention, comprising the steps:

(a) dispersing ethycellulose particles having a volume weight average diameter d4,3 between 50 and 500 nanometer in water at a pH of 4 or lower or at an ionic strength of at least 20 millimolar;
(b) aerating the composition from step (a); and
(c) addition of cationic polymer to the dispersion from step (b).

In this case, the pH of the composition, or the ionic strength of the composition leads to the correct surface charge of the ethylcellulose particles.

Preferably in a further process step (d), addition of triphospate anions, preferably sodium triphosphate ($Na_5P_3O_{10}$), to the composition from step (c).

Preferred embodiments described in the context of the first aspect of the invention, are applicable to the second aspect of the invention, *mutatis mutandis.*

Preferably the aerated composition is used for preparing a food product or a personal care product or a home care product. Preferably the product is made by the method of the invention, further comprising the step (e): mixing the aerated composition from step (c) or (d) with one or more ingredients of a food product;
or one or more ingredients of a personal care product;
or one or more ingredients of a home care product.

In both methods, one or more ingredients for a food product or a personal care product or a home care product may be mixed with the dispersion prior to the aeration step. The order in which the ingredients are brought into contact to make the food product or home care product or personal care product will depend on the product to be prepared. Additionally or alternatively, one or more ingredients for a food product or a personal care product or a home care product may be mixed with the dispersion after the aeration step. In some cases the aerated composition may be mixed with a composition which in itself already forms the food product or home care product or personal care product, albeit in that case without the aerated composition.

## DESCRIPTION OF FIGURES

*Figure 1*: Light microscopy images of foams prepared in example 1, all same scale (bar size 100 micrometer, image width about 600 micrometer)
Left column (marked "1"); top image: foam after 0 min, middle image: foam after 3 min, bottom image: no image)
Middle column (marked "2"): top image: foam after 0 min, middle image: foam after 3 min, bottom image: foam after 20 min)
Right column (marked "3"): top image: foam after 0 min, middle image: foam after 3 min, bottom image: foam after 20 min)
*Figure* 2: Light microscopy images of foams prepared in example 2, all same scale (image width about 600 micrometer)
Left column (marked "4"); top image: foam after 0 min, middle image: foam after 3 min, bottom image: foam after 10 min)
Middle column (marked "5"): top image: foam after 0 min, middle image: foam after 3 min, bottom image: foam after 7 min)
Right column (marked "6"): top image: foam after 0 min, middle image: foam after 3 min, bottom image: foam after 20 min)

## EXAMPLES

The following non-limiting examples illustrate the present invention.

### Materials:

- Ethylcellulose (ethoxyl content 48%) powder was purchased from Sigma-Aldrich (St. Louis, MO, USA). The viscosity of 5 wt% ethylcellulose in the mixture solvent of toluene/ethanol (v/v= 80/20) is 100 cps.
- Food grade ethylcellulose N100 (ethoxyl content 48%-49.5%) was purchased from Hercules (now Ashland Inc., Covington, KY, USA).
- Deionised water (R=18.2 MΩ) was obtained from a Millipore filter system.
- HC, $H_2SO_4$, acetic acid, NaOH, NaCl, KCl and $CaCl_2$ were supplied by Sinopharm Chemical Reagent Co., Ltd (China).
- The polylysine (poly-L-lysine) was received from Beijing's biotech corp. (China), and its molecular weight is about 3000~4000 g/mol.
- Poly(diallyldimethylammonium) (PDDA) (Mw: 10,000~20,000) is purchased from

Sigma-Aldrich (St. Louis, MO, USA).

- Chitosan (75% deacylation) was purchased from Sigma-Aldrich (St. Louis, MO, USA).
- Gelatine (from porcine skin, type A) was purchased from Sigma-Aldrich (St. Louis, MO, USA).
- Gum arabic was obtained from Sinopharm Chemical Reagent Co., Ltd (China).
- Sodium triphosphate, was purchased from Sigma-Aldrich (St. Louis, MO, USA).
- SDS is the surfactant sodium dodecyl sulphate (Sinopharm Chemical Reagent Co., Ltd, China).
- All the chemicals were used as received, unless noted otherwise. All materials are analytical pure grade.
- Mayonnaises and dressings are commercial products of Unilever (Rotterdam, The Netherlands), including Hellmann's Light (23 wt% oil) and Hellmann's real (77 wt% oil), Calvé Half (50 wt% oil) and Calvé extra light (3 wt% oil).

### Equipment and Methodologies

IKA RTC basic magnetic stirrer; KM800 Food mixer (Kenwood major); Ultra-Turrax Homogenizer with an 18mm diameter head; Household mini-mixer was purchased from IKEA shopping mall (Shanghai, China).

*Preparation of ethylcellulose colloidal dispersion*

Ethylcellulose colloidal particles dispersion was obtained from Hunan Kinglong Bio-Resource Co. Ltd. (Changsha, Hunan, China). The preparation was done by slowly adding 45 gram of ethylcellulose powder (ex Ashland) to 3 L

of food grade ethanol (95%) in a vessel under stirring until ethylcellulose was dissolved completely at room temperature (25°C). 12 L of deionised water was quickly added into the above ethylcellulose solution under vigorous stirring, and the mixed ethylcellulose dispersion was obtained after 10 minutes. In total 45 L of the dispersion (3 batches) were transferred to a concentrator with ethanol recovery tank. By tuning the pressure and temperature of the concentrator, most of ethanol was evaporated out at 30°C-40°C, and water was removed at 35-45°C until the volume of dispersion was around 9 L with a ethylcellulose particles content of 1.5 wt%.

To increase the solid content and remove the residual ethanol, the ethylcellulose dispersion was further concentrated using a rotary evaporator (at Unilever R&D, Shanghai, China). Around half of water was evaporated out at 70°C, until the solid content was adjusted to 3.2%. The pH of the dispersion of ethylcellulose was about 6.5 to 6.7. The volume weighted mean diameter d4,3 of the ethylcellulose particles was about 130 nanometer.

*Measurement of ethylcellulose particle size and zeta potential*

The particle size of ethylcellulose dispersion was determined by measuring diluted ethylcellulose particle dispersion (ca. 0.1 % w/w) with a Malvern Zetasizer Nano ZS 2000. The pH dependence of zeta potential and particle size were determined by a titration method: 0.1 wt% ethylcellulose particle dispersion was titrated by adding 0.1 M HCl aqueous solution or 0.1 M NaOH aqueous solution to tune the pH between 2 and 9.

*Preparation of ethylcellulose foams*

Ethylcellulose foams were produced by various foaming techniques such as handshaking, mini-mixer or Ultra-Turrax. Ethylcellulose dispersion (10 ml, 1%) was put into a 25 ml measuring cylinder, and some electrolytes were added, followed by sealing it with Parafilm. The resultant ethylcellulose dispersion was shaken by hand for 2 min. In addition, ethylcellulose foams were produced by shearing ethylcellulose dispersion for 3 min with a mini-mixer or Ultra-Turrax at a desirable shear rate.

The foamability was estimated by measuring the overrun of foam immediately after preparation, while the foam stability was assessed by monitoring foam volume over time at ambient.

*Aeration of mayonnaise with ethylcellulose foams*

After four hours' drainage, the drained water was drawn out by syringe. The ethylcellulose foam was mixed with the desired amount of mayonnaise by hand. In order to prepare massive sample, ethylcellulose foam was mixed with 200 g mayonnaise (degassed by shearing) by a Kenwood mixer at lever 4 for 3 min.

*Stability testing methods:*

Turbiscan (Turbiscan Lab Expert, Formulaction, Toulouse, France): All foams were drained for 3 hours before the drained water was removed by a syringe. A fixed amount of foam was added to each Turbiscan testing bottle respectively. 10ml of 0.5 wt% SDS solution was slowly added to the bottle from the bottom with a syringe, to minimize the impact of SDS on EC bubbles during the process. After that, each sample was measured by Turbiscan immediately.

By adding the SDS the foamed composition was challenged for its foam stability. SDS may act as a destabiliser of the foam, as it may replace the ethylcellulose particles on the gas bubble surface.

*Microscopy*

A small amount of foam was placed onto 0.5% SDS solution on a glass slide, and the visual changes were recorded by light microscopy (DM LB 2, Leica Microsystems Ltd., Germany) subsequently. Also here the SDS may act as foam destabiliser.

**Example 1: Comparison of ethylcellulose foams with and without cationic polymers**

Three foams based on ethylcellulose particles were prepared:

*Foam 1: Ethylcellulose with acetic acid (comparative example)*

20 mL of a 2% ethylcellulose particle dispersion as described above was taken. The absolute value of the zeta-potential of the particles was about 40 mV. The pH was set to 3.4 by dropwise adding acetic acid. The dispersion was homogenized by the Ultra-turrax mixer for 3 minutes at 14,000 rpm, to generate foam. Subsequently, the foam was

drained for 3 hours.

*Foam 2*: *Ethylcellulose with polylysine*

20 mL of a 2% ethylcellulose particle dispersion as described above was taken. The absolute value of the zeta-potential of the particles was about 40 mV. To this 0.14 mL of a 1.46% polylysine (0.1 M polylysine by repeating unit) was added (ratio polylysine:ethylcellulose about 1:200). The absolute value of the zeta-potential of the ethylcellulose particles then was about 15 mV. The mixture was homogenized by the Ultra-Turrax for 3 minutes at 14,000 rpm, to generate foam. Subsequently, the foam was drained for 3 hours.

*Foam 3*: *Ethylcellulose with polyDADMAC*

20 mL of a 2% ethylcellulose particle dispersion as described above was taken. The absolute value of the zeta-potential of the particles was about 40 mV. To this 0.14 mL of a 1.62% polyDADMAC (0.1 M polyDADMAC by repeating unit) was added (ratio polyDADMAC:ethylcellulose about 1:175). The mixture was homogenized by the Ultra-Turrax for 3 minutes at 14,000 rpm, to generate foam. Subsequently, the foam was drained for 3 hours.

To test the robustness of the foams 1, 2, and 3, a sample of each foam was placed on a glass slide containing the surfactant SDS. SDS acts as a destabilizer of the foam, and this challenges the quality of the foam. By following the collapse of the foam, the resistance against SDS can be determined. The results are shown in Figure 1.

Left column (marked "1"); top image: foam after 0 min, middle image: foam after 3 min, bottom image: no image). This shows that the foam with ethylcellulose particles only is very sensitive for SDS, after 3 min. many of the bubbles have reduced in size, and already after 10 min. there are no bubbles left anymore (no image).

Middle column (marked "2"): top image: foam after 0 min, middle image: foam after 3 min, bottom image: foam after 20 min). This shows that the foam with ethylcellulose particles and polylysine is less sensitive for SDS than ethylcellulose only. The bubble size has decreased after 20 min, still many bubbles are present though.

Right column (marked "3"): top image: foam after 0 min, middle image: foam after 3 min, bottom image: foam after 20 min). This shows that the foam with ethylcellulose particles and polyDADMAC is less sensitive for SDS than ethylcellulose only. The bubble size has decreased after 20 min, still many bubbles are present though.

It is concluded that ethylcellulose foam with cationic polymer is more stable than that without cationic polymer.

**Example 2: Comparison of ethylcellulose foams with cationic polymers and sodium triphosphate**

Another three foams based on ethylcellulose particles were prepared:

*Foam 4*: *Ethylcellulose with polylysine and sodium triphosphate*

The pH of 20 mL 2% ethylcellulose dispersion (as described above) was adjusted to around 3.4 by dropwise adding acetic acid, and then the dispersion was homogenized by the Ultra-Turrax for 3 minutes at 14,000 rpm, to generate foam.

Subsequently, 2.48 mL of 1.46% polylysine solution was added under hand-stirring. Then 24 mL of 0.5% sodium triphosphate solution was added until the mixture became homogenous. The foam was drained for 3 hours.

Weight ratio polylysine : sodium triphosphate : ethylcellulose was about 18 : 60 : 200 (weight ratio polylysine : triphosphate : ethylcellulose was about 20:56:200).

*Foam 5*: *Ethylcellulose with polyDADMAC and sodium triphosphate*

The pH of 20 mL 2% ethylcellulose dispersion (as described above) was adjusted to around 3.5 by dropwise adding acetic acid, and then the dispersion was homogenized by the Ultra-Turrax for 3 minutes at 14,000 rpm, to generate foam.

Subsequently, 2.48 mL of 1.62% polyDADMAC solution was added under hand-stirring. Then 24 mL of 0.5% sodium triphosphate solution was added until the mixture became homogenous. The foam was drained for 3 hours.

Weight ratio polyDADMAC : sodium triphosphate : ethylcellulose was about 20:60:200 (meaning weight ratio polyDADMAC : triphosphate : ethylcellulose was about 20:56:200).

*Foam 6*: *Ethylcellulose with chitosan and sodium triphosphate*

The pH of 20 mL 2% ethylcellulose dispersion (as described above) was adjusted to around 3.5 by dropwise adding acetic acid, and then the dispersion was homogenized by the Ultra-Turrax for 3 minutes at 14,000 rpm, to generate

foam.

Subsequently, 4 mL of 1% chitosan solution containing 1% acetic acid (pH about 3) was added. Then 24 mL of 0.5% sodium triphosphate solution was added until the mixture became homogenous. The foam was drained for 3 hours.

Weight ratio chitosan : sodium triphosphate : ethylcellulose was about 20:60:200 (meaning weight ratio chitosan : triphosphate : ethylcellulose was about 20:56:200). To test the robustness of the foams 4, 5, and 6, a sample of each foam was placed on a glass slide containing the surfactant SDS. SDS acts as a destabilizer of the foam, and this challenges the quality of the foam. By following the collapse of the foam, the resistance against SDS can be determined. The results are shown in Figure 2.

Left column (marked "4"); top image: foam after 0 min, middle image: foam after 3 min, bottom image: foam after 10 min). This shows that ethylcellulose combined with polylysine and triphosphate creates a very stable foam, that is stable against challenge using SDS.

Middle column (marked "5"): top image: foam after 0 min, middle image: foam after 3 min, bottom image: foam after 7 min). This shows that ethylcellulose combined with polyDADMAC and triphosphate creates a foam that is more stable than foam 1 (ethylcellulose only).

Right column (marked "6"): top image: foam after 0 min, middle image: foam after 3 min, bottom image: foam after 20 min). This shows that ethylcellulose combined with chitosan and triphosphate creates a very stable foam, that is stable against challenge using SDS.

**Example 3: Aerated Mayonnaise with ethylcellulose - chitosan - sodium tripolyphosphate foam**

A foam like foam 6 in example 2 was prepared, with 100 mL of a 2% ethylcellulose dispersion as basis. Chitosan (concentration 1% at pH 3 (1 % acetic acid)) and sodium triphospate (at 0.5% concentraion) were added, at various amounts. The foam was mixed with 100 g mayonnaise, Hellmann's Real Mayonnaise (77 wt% of oil). In the final aerated mayonnaise, air fraction was about 30 vol%, meaning the overrun was about 40%.

The stability of the foams was followed by determining the average foam bubble diameter using Turbiscan. The ratio of the gas bubble diameter at time t [d(t)] and time 0 [d(0)] (directly after production) was determined, and the closer that ratio is to 1, the better the bubble stability is. During storage of the aerated mayonnaises, the bubble diameter generally increases.

The results of these experiments are indicated in the following.

*Table 1 Influence of weight ratio ethylcellulose : chitosan ("EC:chit", chitosan $M_w$ 250, 000 g/mol) on bubble size during storage, at a fixed weight ratio of chitosan : sodium triphosphate of 1:3.*

| *Time [day]* | *Ratio d(t)/d(0)* | | | | | |
|---|---|---|---|---|---|---|
| | *EC only (no chitosan)* | *ratio EC: chit 40:1* | *ratio EC: chit 20:1* | *ratio EC: chit 10:1* | *ratio EC: chit 5:1* | *ratio EC: chit 3:1* |
| 0 | 1 | 1 | 1 | 1 | 1 | 1 |
| 20 | 1.5 | 1.35 | 1.2 | 1.15 | 1.4 | 1.33 |
| 40 | 1.6 | 1.38 | 1.25 | 1.15 | 1.42 | 1.35 |
| 60 | 1.7 | 1.4 | 1.3 | 1.18 | 1.5 | 1.4 |
| 80 | 1.8 | 1.4 | 1.35 | 1.25 | 1.5 | 1.4 |
| 100 | 1.9 | 1.5 | 1.35 | 1.28 | 1.6 | 1.46 |
| 120 | 2.05 | 1.6 | 1.4 | 1.3 | 1.6 | 1.5 |
| 140 | 2.1 | 1.6 | 1.4 | 1.3 | 1.62 | 1.5 |

This table shows that the addition of chitosan leads to improved stability of the foam, as compared to a foam with EC only.

The influence of the molecular weight can be shown by the following measurement data, with a weight ratio of ethylcellulose : chitosan : sodium triphosphate of 10:1:3.

Chitosan $M_w$ 50,000 g/mol: d(100 days)/d(0) = 1.5
Chitosan $M_w$ 100,000 g/mol: d(100 days)/d(0) = 1.4
Chitosan $M_w$ 250,000 g/mol: d(100 days)/d(0) = 1.28

These data show that increasing molecular weight improves stability of the aerated mayonnaise.

*Table 2 Influence of weight ratio ethylcellulose : chitosan : sodium triphospate ("EC: chit: STP') on bubble size during storage, with Chitosan of $M_w$ 250, 000 g/mol.*

| *Time [day]* | *Ratio d(t)/d(0)* | | | | |
|---|---|---|---|---|---|
| | *EC only (no chitosan)* | *ratio EC:chif:STP 10:1:0.5* | *ratio EC:chit:STP 10:1:1* | *ratio EC:chif:STP 10:1:2* | *ratio EC:chif:STP 10:1:3* |
| 0 | 1 | 1 | 1 | 1 | 1 |
| 20 | 1.5 | 1.2 | 1.15 | 1.12 | 1.05 |
| 40 | 1.6 | 1.23 | 1.2 | 1.2 | 1.12 |
| 60 | 1.7 | 1.25 | 1.22 | 1.18 | 1.13 |
| 80 | 1.8 | 1.28 | 1.22 | 1.22 | 1.16 |
| 100 | 1.9 | 1.3 | 1.24 | 1.24 | 1.17 |
| 120 | 2.05 | 1.35 | 1.25 | 1.24 | 1.19 |

These data show that increased sodium triphosphate content leads to improved stability of the foam.

**Example 4: Aerated Mayonnaise with ethylcellulose - gelatin foams**

A foam was prepared, with 100 mL of a 2% ethylcellulose dispersion as basis, and adjusted to pH 3.4 with acetic acid. Gelatin type A was added. The foaming procedure was similar as in examples 1 and 2. The obtained foams was mixed with 100 g Hellmann's real mayonnaise. In the final aerated mayonnaise, air fraction was about 30 vol%, meaning the overrun was about 40%.

The stability of the foams was followed by determining the average foam bubble diameter using Turbiscan. The ratio of the gas bubble diameter at time t [d(t)] and time 0 [d(0)] (directly after production) was determined, and the closer that ratio is to 1, the better the bubble stability is. During storage of the aerated mayonnaises, the bubble diameter generally increases.

Various concentrations of gelatin were tested. When the ratio ethylcellulose to gelatin was larger than 20, the stability of the system at 100 days was better than the similar system without ethylcellulose.
Ratio EC:gelatin = 20:1. d(100 days)/d(0) = 1.8
Ratio EC:gelatin = 100:1. d(100 days)/d(0) = 1.75
EC only: d(100 days)/d(0) = 1.9

The influence of molecular weight of gelatin was shown, by testing gelatin types with different gel strengths: 100, 180, and 220 bloom respectively. This relates to short, medium, and high molecular weight, respectively. In short the results are the following:

*Table 3 Influence of gel strength of gelatin on foam stability (weight ratio ethylcellulose : gelatin = 100:1)*

| *Time [day]* | *Ratio d(t)/d(0)* | | | |
|---|---|---|---|---|
| | *No gelatin (EC only)* | *Gelatin low $M_w$, 100 bloom* | *Gelatin medium $M_w$, 180 bloom* | *Gelatin high $M_w$ 220 bloom* |
| 0 | 1 | 1 | 1 | 1 |
| 20 | 1.5 | 1.5 | 1.5 | 1 |
| 40 | 1.6 | 1.7 | 1.55 | 1.3 |
| 60 | 1.7 | 1.8 | 1.6 | 1.3 |
| 80 | 1.8 | 1.8 | 1.6 | 1.3 |
| 100 | 1.9 | 1.8 | 1.6 | 1.4 |
| 120 | 2.0 | 2 | 1.7 | 1.4 |

This shows that especially the medium and high molecular weight show good results on stability.

In a next set of experiments gum arabic was added to the foam, before being mixed with mayonnaise. This led to strongly increased stability of the aerated mayonnaise.

*Table 4 Influence of gum arabic and gelatin (with high molecular weight, 220 bloom) on foam stability (different weight ratio ethylcellulose : gelatin : gum arabic "EC:gelat:gum ar")*

| Time [day] | Ratio d(t)/d(0) | | |
|---|---|---|---|
| | No gelatin (EC only) | Ratio EC:gelat:gum ar 8:1:1 | Ratio EC:gelat:gum ar = 2:1:1 |
| 0 | 1 | 1 | 1 |
| 20 | 1.5 | 1.3 | 1.2 |
| 40 | 1.6 | 1.3 | 1.4 |
| 60 | 1.7 | 1.25 | 1.45 |
| 80 | 1.8 | 1.3 | 1.5 |
| 100 | 1.9 | 1.55 | 1.3 |
| 120 | 2.0 | 1.6 | 1.55 |
| 140 | 2.1 | 1.65 | 1.5 |

This shows that addition of gum arabic leads to a strongly increased stability of the foam stabilised by ethylcellulose and gelatin.

## Claims

1. An aqueous aerated composition comprising ethylcellulose particles and a cationic polymer,
wherein the ethylcellulose particles have a volume weight average diameter d4,3 between 50 and 500 nanometer, and wherein at least part of the ethylcellulose particles and cationic polymer is present at the gas bubble surface.

2. A composition according to any of claim 1, wherein the ethylcellulose particles have a volume weight average diameter d4,3 between 50 and 400 nanometer.

3. A composition according to any of claims 1 to 2, wherein at least 95% of the ethylcellulose particles has a diameter smaller than 500 nanometer, preferably smaller than 250 nanometer.

4. A composition according to any of claim 1 to 3, having an overrun of at least 1%, preferably at least 5%.

5. A composition according to any of claims 1 to 4, wherein the absolute value of the zeta-potential of the ethylcellulose particles is below 25 millivolt.

6. A composition according to any of claims 1 to 5, wherein the concentration of ethylcellulose particles ranges from 0.5% to 10% by weight, preferably from 1% to 5% by weight.

7. A composition according to any of claims 1 to 6, wherein the cationic polymer is chosen from the group consisting of poly-L-lysine, polydiallyldimethylammonium chloride, chitosan and gelatin.

8. A composition according to any of claims 1 to 7, wherein the concentration of polymer ranges from 0.005% to 5% by weight of the composition.

9. A composition according to any of claims 1 to 8, further comprising triphospate anions ($P_3O_{10}^{5-}$), preferably sodium triphosphate ($Na_5P_3O_{10}$).

10. A composition according to any of claims 1 to 7, wherein the cationic polymer comprises gelatin, and the composition further comprises gum arabic.

**11.** A food product or a personal care product or a home care product comprising the composition according to any of claims 1 to 10.

**12.** A method for preparation of a composition according to any of claims 1 to 11, comprising the steps:

(a) dispersing ethylcellulose particles having a volume weight average diameter d4,3 between 50 and 500 nanometer in an aqueous composition;
(b) addition of cationic polymer to the dispersion from step (a); and
(c) aerating the composition from step (b).

**13.** A method for preparation of a composition according to any of claims 1 to 11, comprising the steps:

(a) dispersing ethycellulose particles having a volume weight average diameter d4,3 between 50 and 500 nanometer in water at a pH of 4 or lower or at an ionic strength of at least 20 millimolar;
(b) aerating the composition from step (a); and
(c) addition of cationic polymer to the dispersion from step (b).

**14.** A method according to claim 12 or 13, further comprising the step:

(d) addition of triphospate anions, preferably sodium triphosphate ($Na_5P_3O_{10}$), to the composition from step (c).

**15.** A method according to any of claims 12 to 14, further comprising the step:

(e) mixing the aerated composition from step (c) or (d) with one or more ingredients of a food product;
or one or more ingredients of a personal care product;
or one or more ingredients of a home care product.

Figure 1

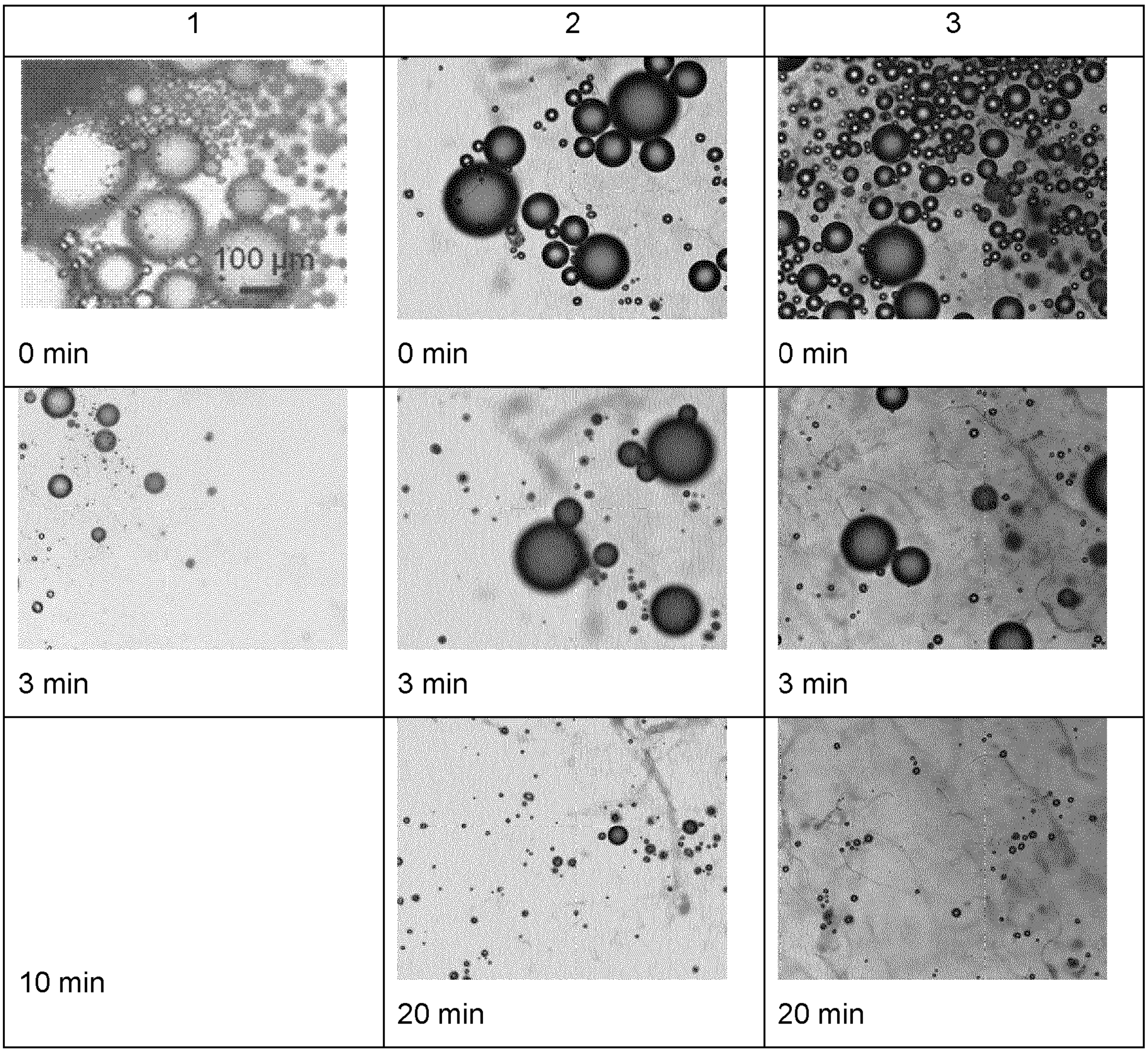

Figure 2

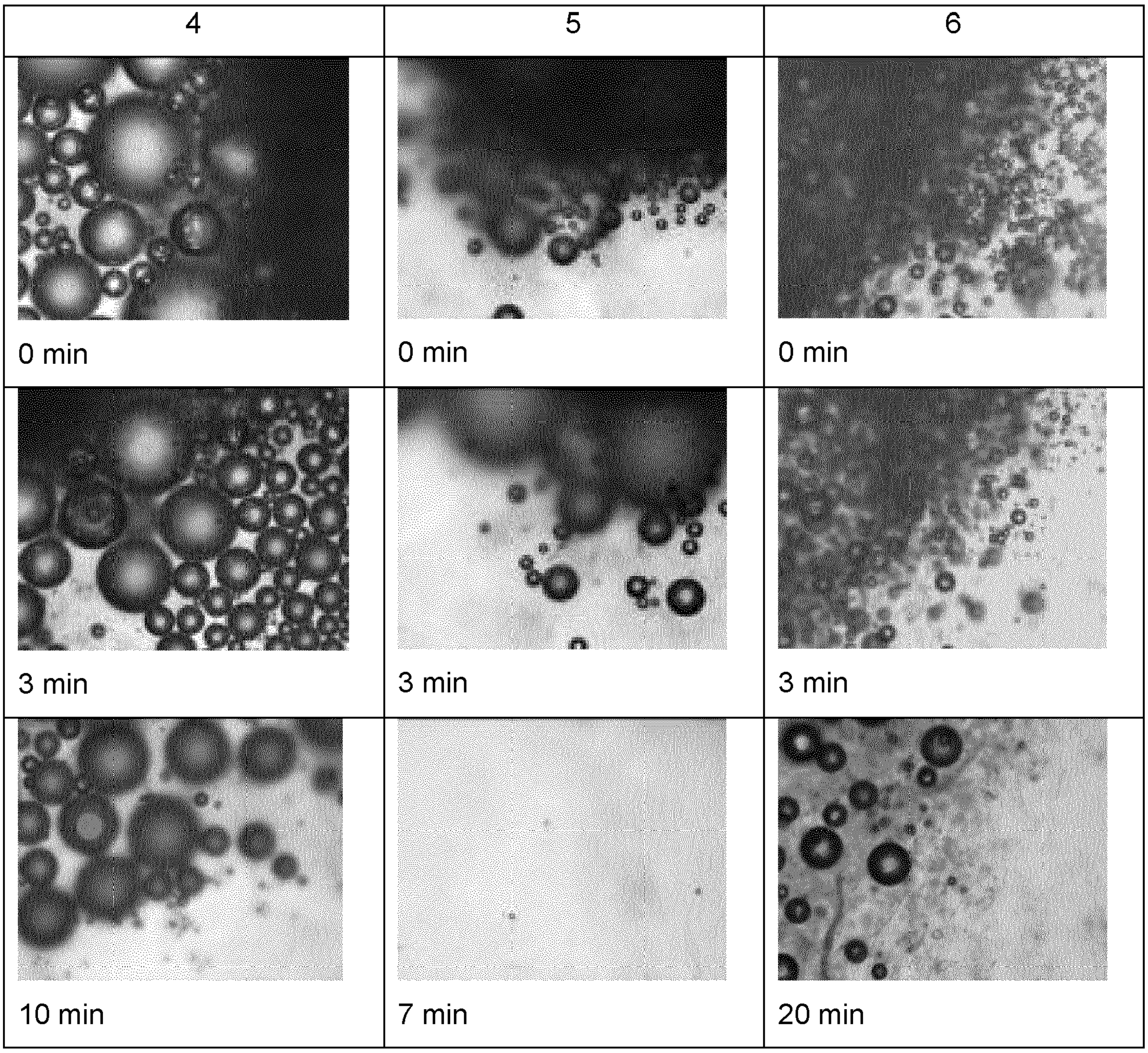

Europäisches Patentamt
European Patent Office
Office européen des brevets

# EUROPEAN SEARCH REPORT

Application Number
EP 12 15 5410

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | DE 10 2007 043299 A1 (BIOZOON FOOD INNOVATIONS GMBH [DE]) 12 March 2009 (2009-03-12) * paragraph 0009; claims 1-23 * ----- | 1-15 | INV. A23L1/00 A23L1/03 A23L1/0534 A61K8/00 C11D17/00 |
| A,D | WO 2008/046699 A1 (UNILEVER NV [NL]; UNILEVER PLC [GB]; UNILEVER HINDUSTAN [IN]; BLIJDENS) 24 April 2008 (2008-04-24) * page 6, lines 14-18; claims 1-31 * ----- | 1-15 | |
| A | WO 2011/015444 A1 (UNILEVER NV [NL]; UNILEVER PLC [GB]; UNILEVER HINDUSTAN [IN]; ARNAUDOV) 10 February 2011 (2011-02-10) * page 3, lines 18-27; claims 1-8 * ----- | 1-15 | |
| | | | TECHNICAL FIELDS SEARCHED (IPC) |
| | | | A23L A61K C11D |

The present search report has been drawn up for all claims

1

| Place of search | Date of completion of the search | Examiner |
|---|---|---|
| Munich | 2 May 2012 | Georgopoulos, N |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 12 15 5410

This annex lists the patent family members relating to the patent documents cited in the above-mentioned European search report.
The members are as contained in the European Patent Office EDP file on
The European Patent Office is in no way liable for these particulars which are merely given for the purpose of information.

02-05-2012

| Patent document cited in search report | Publication date | Patent family member(s) | Publication date |
|---|---|---|---|
| DE 102007043299 A1 | 12-03-2009 | DE 102007043299 A1 | 12-03-2009 |
| | | WO 2009033592 A1 | 19-03-2009 |
| WO 2008046699 A1 | 24-04-2008 | AU 2007312500 A1 | 24-04-2008 |
| | | CA 2665925 A1 | 24-04-2008 |
| | | EP 2081443 A1 | 29-07-2009 |
| | | JP 2010506576 A | 04-03-2010 |
| | | RU 2009118469 A | 27-11-2010 |
| | | US 2010189857 A1 | 29-07-2010 |
| | | WO 2008046699 A1 | 24-04-2008 |
| WO 2011015444 A1 | 10-02-2011 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008019865 A1
- WO 2008046699 A1
- WO 2009033592 A1
- WO 2006067064 A1
- WO 2007038745 A1
- WO 2010121490 A1


### Non-patent literature cited in the description


- **PLASARI et al.** *Trans IChemE,* 1997, vol. 75, 237-244
- **B.S. MURRAY.** *Food Hydrocolloids,* 2011, vol. 25, 627-638